# EUROPEAN PATENT APPLICATION

(11) **EP 4 385 982 A1**
(43) Date of publication of application: **19.06.2024**
(21) Application number: 22214079.0
(22) Date of filing: 16.12.2022
(51) Int. Cl.: C07D 335/16, C08F 293/00, C08L 23/08

(54) **METHOD OF PREPARING A MONOMERIC PHOTOINITIATOR**

(71) Applicant: Henkel AG & Co. KGaA, 40589 Düsseldorf (DE)
(72) Inventor: Arnold, Moritz, 41836 Hückelhoven (DE); Taden, Andreas, 40597 Düsseldorf (DE); Schneider, Anja, 40547 Düsseldorf (DE)

(57) **Abstract**

The present disclosure provides a method of preparing a compound of Formula (V) wherein n of radicals R ^{6'} to R ^{9'} are -R ^{b} OC(O)C(R ¹³ )=CH ₂ , and further wherein R ¹³ is H or C ₁ alkyl, n is an integer of from 1 to 3 and for each of said n radicals, R ^{b} is independently selected from a covalent bond, C ₂ -C ₁₂ alkylene, C ₃ -C ₁₈ cycloalkylene or C ₆ -C ₁₈ arylene. In a first method step i), a disulfide compound of Formula (I) is reacted in an acidic medium having a pH of ≤ 4 with a hydroxyl functional compound of Formula (II) to yield a compound of Formula (III), wherein: R ² to R ⁵ are independently selected from H or C ₁ -C ₆ alkyl, R ⁶ to R ⁹ correspond to said substituents R ^{6'} to R ^{9'} and are independently selected from H, C ₁ -C ₆ alkyl, C ₁ -C ₆ alkoxy, C ₁ -C ₁₂ alkoxyalkyl, SR ¹² , COOR ¹² and N(R ¹² ) ₂ ; R ¹⁰ and R ¹¹ are H; and, each R ¹² is independently selected from C ₁ -C ₆ alkyl or C ₆ -C ₁₈ aryl, subject to the proviso that n of radicals R ⁶ to R ⁹ are R ^{b} (OH) and for each of said n radicals, R ^{b} is independently selected as defined above. In a second step ii) said compound of Formula (III) is reacted in an inert aprotic solvent with a (meth)acrylating agent of Formula (IV) to yield said compound of Formula (V), wherein: in Formula (IV), X is halide or -OC(O)C(R ¹³ )=CH ₂ ; and, the number of moles of (meth)acrylate groups provided by said compound of Formula (IV) is at least equimolar with the number of moles of hydroxyl groups provided by the compound of Formula (III).

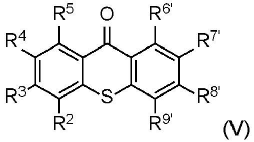

## Description

### FIELD OF THE INVENTION

The present disclosure is directed to a method of preparing a compound having an ethylenically unsaturated group and a moiety that is decomposable under photo-irradiation to form a radical. The compound may have utility in the preparation of a copolymer having pendant photoreactive groups.

### BACKGROUND TO THE INVENTION

The preparation of compositions - such as but not limited to reactive hot melt compositions - which may be cured upon photo-irradiation and, in particular, under ultraviolet light is known in the art. Broadly, photocuring offers flexibility as a crosslinking method since the user can determine the position and time at which photo-irradiation occurs and can moderate the exposure of the curable composition to that irradiation.

Photocuring compositions will typically comprise one or more photo-initiators. The term *"photoinitiator"* as used herein denotes a compound which can be activated by an energy-carrying activation beam - such as electromagnetic radiation - upon irradiation therewith. The present disclosure concerns a *"free-radical photoinitiator"* which herein refers to a photoactive compound that generates free radicals.

Free-radical photoinitiators are conventionally categorized into Norrish type I and Norrish type II photoinitiators. A Norrish type I radical photoinitiator undergoes the Norrish type I reaction when exposed to actinic radiation: said reaction is defined by IUPAC as α-cleavage of an excited carbonyl compound leading to an acyl-alkyl radical pair (from an acyclic carbonyl compound) or an acyl-alkyl biradical (from a cyclic carbonyl compound) as a primary photoproduct. A Norrish type II radical photoinitiator undergoes the Norrish type II reaction when exposed to actinic radiation: that reaction is defined by IUPAC as the photochemical abstraction of a γ-hydrogen by an excited carbonyl compound to produce a 1 ,4-biradical as a primary photoproduct.

Low molecular photo-initiators - having, for instance, a molecular weight of less than 1000 Daltons - which are not reacted in the curing process can move diffusively within cured films. This transition of photo-initiators is referred to as migration and can be deleterious to most applications of cured films. It is particularly so with regard to food packaging applications, where the permissible level of migration of low molecular weight compounds - such as said photo-initiators and their degradation products but also including *inter alia* unreacted monomers, photosensitizers, stabilizers and scavengers - from packaging films and associated printed articles to food stuffs is the subject of legislative controls. EU Regulations 1935/2004 and 2020/1245, for example, detail that the specific migration value (SML) of non-genotoxic low molecular weight compounds from articles intended to come into contact with food is 10 micrograms per kilogram of food.

The tendency of Norrish Type II (*hydrogen abstraction*) photo-initiators to migrate or be extracted from cured poly(meth)acrylate films is, in theory, higher than for Norrish Type I (*cleavage*) photo-initiators. Norrish type I photo-initiators generate two highly reactive free radicals which tend to be bound into the cured film by reacting with an acrylate group. Norrish Type II hydrogen abstraction photoinitiators also produce two free radicals in a bimolecular reaction with an amine synergist. Of these, the aminoalkyl radical is highly reactive and binds into the cured film by reacting with an acrylate group, but the ketyl radical has a low reactivity towards acrylate bonds and undergoes termination reactions, or else oxidises back to the ketone. Solvent extraction of a cured film never recovers all of the Norrish Type II photo-initiator.

In certain circumstances, the migration of Norrish Type II photo-initiators has been mitigated in the art by using multifunctional rather than mono-functional initiators which can become bound within the cured films. Minimizing the initial concentration of photo-initiators in the curable compositions is an alternative strategy but one which relies upon the efficiency with which the photo-initiators convert the energy of the irradiation source. As a further alternative, copolymers having pendant photoreactive groups have been developed in the art.

WO2021/225778 A1 (Henkel IP and Holding GmbH) describes a photo-crosslinker which is responsive to ultraviolet light having a wavelength of 365 nm or higher, said photo-crosslinker having a structure as defined in Formula (I) below: 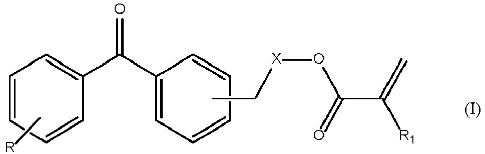 wherein:
R is H, C₁ to C₃₀ alkyl, C₁ to C₃₀ alkoxy, C₁ to C₃₀ acyloxy, C₃ to C₃₀ aryloxy, halogen or C₁ to C₃₀ thioether;
R¹ is H or CH₃; and,
X is optional, when X is not present R is not H.

Further is disclosed a hot melt pressure sensitive adhesive comprising a (meth)acrylate polymer having incorporated therein the photo-crosslinker according to Formula (I).

EP-A-0 017 364 (Rohm & Haas) describes copolymers which may be used in *inter alia* adhesives and sealants, which copolymers comprise from 0.1 to 10 wt.% by weight of allylbenzoyl benzoate as a co-polymerized photoinitiator. Although these materials can be crosslinked using UV radiation, it is considered that their reactivity towards such radiation is too low, leading to low curing efficiency particularly at deeper point in layers of the material. Moreover, layers produced from the copolymers are not considered sufficiently tacky for certain adhesive applications.

The low reactivity and inefficiency of crosslinking copolymers comprising from 0.01 to 5 wt.% of co-polymerizable 2-alkoxy-2-phenyl-2-benzoylethyl acrylate is also considered a disadvantage of the teaching of US Patent No. 4,144,157 (Beiersdorf AG).

In practice, the low curing efficiency of copolymers comprising co-polymerizable photo-initiators might be moderated by increasing the dosage of the applied irradiation. However, low curing efficiency has contributed, unfortunately, to the sustained use of mercury-based UV systems for the photo-irradiation of cross-linking polymers: mercury lamps provide a broad band spectral distribution such that shorter wavelength light promotes surface cure of the applied compositions, whilst longer wavelength light effects deeper cure.

These solutions may not however be desirable or, indeed, viable in certain applications. In particular, following the 2013 Minamata Convention on Mercury, the manufacture, import or export of mercury lamps became illegal in January 2020. There is therefore a need in the art to economically provide copolymers comprising co-polymerizable photoinitiators which are responsive to alternative sources of UV-irradiation to mercury lamps, at both practicable coating weights and dosages of the applied irradiation. It would certainly be advantageous to economically provide copolymers comprising co-polymerizable photoinitiators which can be cured using UV Light Emitting Diodes (UV-LEDs) given that such systems present the advantages of *inter alia*: small size; lack of fragility; temperature-independent output; and, the absence of lead or warm-up time.

Thioxanthone and derivatives thereof have been identified as potentially valuable photo-initiators on the basis that they show two absorption peaks in the ultraviolet region of the electromagnetic spectrum. CN200410093977 (Jiuri Chemical Co. Ltd) and WO2012/003644 A1 (Tianjin Jiuri Chemical Co. Ltd), for example, describes thioxanthone-4-carboxylates, their preparation methods and their utility in photo-initiator compositions. Further, US Patent No. 7,354,957 (Herlihy) discloses a compound of Formula (I) which has utility as a photo-initiator: 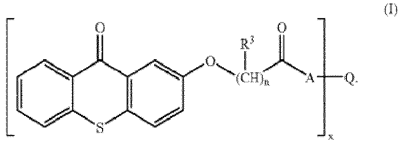 wherein n is a number from 1 to 6; R³ is hydrogen, methyl or ethyl: A represents a group of formula -[O(CHR²CHR¹)ₐ]_{y}-, -[O(CH₂)_{b}CO)]_{y}-, -O(CH₂)_{b}CO)]_{y}, or -[O(CHR²CHR¹)a]-, where one of R' and R² is hydrogen and the other is hydrogen, methyl or ethyl; a is 1 or 2; b is 4 or 5; y is from 3 to 10; Q is a residue of a polyhydroxy compound having 2 to 6; and, X is greater than 1.

U.S. Pat. No. 5,248,805 (Boettcher et al.) describes thioxanthone derivatives having a spacer group, such as a carbonate group, to connect the thioxanthone sensitizer to an ethylenically unsaturated group.

The present inventors have recognized a need in the art to develop a practicable method for economically and efficiently providing thioxanthone derivatives which may be co-polymerizable with ethylenically unsaturated monomers to form a copolymer.

### STATEMENT OF THE INVENTION

In accordance with a first aspect of the disclosure, there is provided a method of preparing a compound of Formula (V): 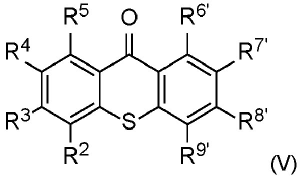 wherein: R^{6'} to R^{9'} are independently selected from H, C₁-C₆ alkyl, C₁-C₆ alkoxy, C₁-C₁₂ alkoxyalkyl, SR¹², COOR¹² and N(R¹²)₂; and,
each R¹² is independently selected from C₁-C₆ alkyl or C₆-C₁₈ aryl,
subject to the proviso that n of radicals R^{6'} to R^{9'} are -R^{b}OC(O)C(R¹³)=CH₂ wherein:
   R¹³ is H or C₁ alkyl;
   n is an integer of from 1 to 3, preferably 1 or 2; and,
   for each of said n radicals, R^{b} is independently selected from a covalent bond, C₂-C₁₂ alkylene, C₃-C₁₈ cycloalkylene or C₆-C₁₈ arylene,
said method comprising the steps of:
i) reacting a disulfide compound of Formula (I) with a hydroxyl functional compound of Formula (II) to yield a compound of Formula (III) 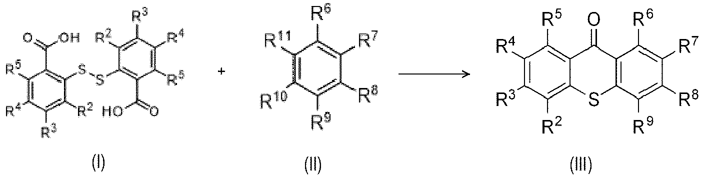 wherein: said reaction is performed in an acidic medium having a pH at or below 4.0;
   R² to R⁵ are independently selected from H or C₁-C₆ alkyl;
   R⁶ to R⁹ correspond to said substituents R^{6'} to R^{9'} and are independently selected from H, C₁-C₆ alkyl, C₁-C₆ alkoxy, C₁-C₁₂ alkoxyalkyl, SR¹², COOR¹² and N(R¹²)₂;
   R¹⁰ and R¹¹ are H; and,
   each R¹² is independently selected from C₁-C₆ alkyl or C₆-C₁₈ aryl,
   subject to the proviso that n of radicals R⁶ to R⁹ are R^{b}(OH) wherein, for each of said n radicals, R^{b} is independently selected from a covalent bond, C₂-C₁₂ alkylene, C₃-C₁₈ cycloalkylene or C₆-C₁₈ arylene; and,
ii) reacting in an inert aprotic solvent said compound of Formula (III) with a compound of Formula (IV) to yield said compound of Formula (V) 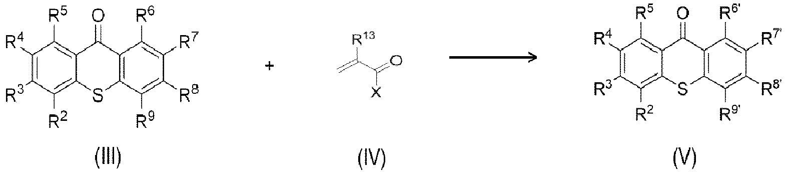 wherein: X is halide or -OC(O)C(R¹³)=CH₂; and,
   the number of moles of (meth)acrylate groups provided by said compound of Formula (IV) is at least equimolar with the number of moles of hydroxyl groups provided by the compound of Formula (III).

As regards said Formula (I), it is preferred that R² to R⁵ are independently selected from H or C₁-C₄ alkyl. For example, R² to R⁵ may be independently selected from H or C₁-C₂ alkyl and, in some embodiments, R² to R⁵ are each H.

As regards said Formula (II), it is preferred that R⁶ to R⁹ are independently selected from H, C₁-C₄ alkyl, C₁-C₄ alkoxy, C₁-C₈ alkoxyalkyl, COOR¹² and N(R¹²)₂; R¹⁰ and R¹¹ are H; and, each R¹² is independently selected from C₁-C₄ alkyl or C₆-C₁₈ aryl, subject to the proviso that n of radicals R⁶ to R⁹ are -OH, wherein n is an integer of 1 or 2.

Step i) of the process may, in certain embodiments, be performed in an acidic medium having a pH at or below 3.5, for example at or below 3.0. Alternatively or additionally to this statement of pH conditions, step ii) should desirably be performed at a temperature below the boiling point of the acid medium: subject to meeting that requirement, step i) may be performed at a temperature of from 20°C to 120°C, for example from 40 to 120°C or from 60 to 100°C.

In step ii), the molar ratio of (meth)acrylate groups provided by said compound of Formula (IV) to hydroxyl groups provided by said compound of Formula (III) should typically be from 1:1 to 2:1, preferably from 1.1:1 to 1.5:1 and more preferably from 1.1:1 to 1.4: 1. Independently of or additional to these preferred statements of molar ratio, step ii) should be performed at a temperature of from -40 to 20°C, preferably from -20 to 20°C.

As regards said Formula (IV), it desirable that X is halide and preferred that X is chloride. When X is a halide, the reaction of step ii) should be performed in the presence of a base such as, but not limited to, tertiary amine. For example, said tertiary amine base may be selected from the group consisting of: tri(C₁-C₁₂)alkyl amines; di(C₁-C₁₂)alkyl(C₃-C₈)cycloalkyl amines; tri(C₁-C₁₀)alkenyl amines; and, mixtures thereof. And exemplary tri(C1-C12)alkyl amines, which may be included alone or in combination, include: trimethylamine; ethyldimethylamine; diethylmethylamine; triethylamine; triisopropylamine; tri-n-propylamine; tri-n-butylamine; tri-sec-butylamine; dibutylpentylamine; n-butyl-octyl-sec-butylamine; tripentylamine; trihexylamine; and, mixtures thereof.

In accordance with a second aspect of the present disclosure there is provided the use of a compound of Formula (V) obtained in accordance with the method as defined herein above and the appended claims as a monomer in a free-radical polymerization.

The disclosure further provides for a copolymer obtained by free radical polymerization, said copolymer comprising, based on the total weight of monomers: from 0.1 to 10 wt.% of a) at least one compound of Formula (V) obtained in accordance with the method as defined herein above and in the appended claims; and, from 90 to 99.9 wt.% of b) at least one ethylenically unsaturated non-ionic monomer which does not bear an epoxide group or a moiety decomposable under photo-irradiation to form a radical.

### DEFINITIONS

As used herein, the singular forms "*a*", *"an"* and *"the"* include plural referents unless the context clearly dictates otherwise.

The terms *"comprising", "comprises"* and *"comprised of*" as used herein are synonymous with *"including", "includes", "containing"* or *"contains"*, and are inclusive or open-ended and do not exclude additional, non-recited members, elements or method steps.

As used herein, the term *"consisting of"* excludes any element, ingredient, member or method step not specified. For completeness, the term *"comprising"* encompasses *"consisting of*"*.*

When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

Further, in accordance with standard understanding, a weight range represented as being *"from 0 to x"* specifically includes 0 wt.%: the ingredient defined by said range may be absent from the material or may be present in the material in an amount up to x wt.%.

The words *"preferred", "preferably", "desirably"* and *"particularly"* are used frequently herein to refer to embodiments of the disclosure that may afford particular benefits, under certain circumstances. However, the recitation of one or more preferable, preferred, desirable or particular embodiments does not imply that other embodiments are not useful and is not intended to exclude those other embodiments from the scope of the disclosure.

The word *"exemplary"* is used herein to mean serving as an example, instance, or illustration. Any aspect or design described herein as *"exemplary"* is not necessarily to be construed as preferred or advantageous over other aspects or designs. Rather, use of the word exemplary is intended to present concepts in a concrete fashion.

As used throughout this application, the word *"may"* is used in a permissive sense - that is meaning to have the potential to - rather than in the mandatory sense.

As used herein, room temperature is 23°C plus or minus 2°C.

The molecular weights referred to in this specification can be measured with gel permeation chromatography (GPC) using polystyrene calibration standards, such as is done according to ASTM 3536.

The term *"aprotic solvents"* as used herein refers to solvents that do not yield or accept a proton. Conversely *"protic solvents"* are those solvents capable of yielding or accepting a proton. The *"polar solvent"* as used herein refers to a solvent having a dielectric constant (ε) of more than 5 as measured at 25°C: the term encompasses both aprotic and protic solvents. The determination of dielectric constant (ε) is well known in the art and is within the knowledge of the skilled person: the use of measured voltages across parallel plate capacitors in such determinations may be mentioned.

As used herein, the term "*free radical initiator"* refers to any chemical species which, upon exposure to sufficient energy - in the form of light or heat, for example - decomposes into two parts which are uncharged, but which each possess at least one unpaired electron. Thus a thermal free radical initiator generates free upon exposure to heat. And known thermal free radical initiators include, but are not limited to, peroxide compounds, azo compounds and persulfate compounds.

As used herein, the term *"monomer"* refers to a substance that can undergo a polymerization reaction to contribute constitutional units to the chemical structure of a polymer. The term "*monofunctional*"*,* as used herein, refers to the possession of one polymerizable moiety. The term *"polyfunctional",* as used herein, refers to the possession of more than one polymerizable moiety.

As used herein, the term *"water"* is intended to encompass tap water, spring water, purified water, deionized water, de-mineralized and distilled water.

As used herein *"solvents"* are substances capable of dissolving another substance to form a uniform solution; during dissolution neither the solvent nor the dissolved substance undergoes a chemical change. Solvents may either be polar or non-polar.

As used herein, the term *"disulfide group"* refers to a functional group with the structure R-S-S-R'. Where a compound is referred to a being a disulfide, the compound must possess an -S-S-linkage.

As used herein, "*(meth)acryl*" is a shorthand term referring to *"acryl"* and/or *"methacryl".* Thus, the term *"(meth)acrylamide"* refers collectively to acrylamide and methacrylamide.

As used herein, *"C₁-Cₙ alkyl"* group refers to a monovalent group that contains 1 to n carbons atoms, that is a radical of an alkane and includes straight-chain and branched organic groups. As such, a *"C₁-C₁₈ alkyl"* group refers to a monovalent group that contains from 1 to 18 carbons atoms, that is a radical of an alkane and includes straight-chain and branched organic groups. In general, a preference for alkyl groups containing from 1-12 carbon atoms (C₁-C₁₂ alkyl) - for example alkyl groups containing from 1 to 8 carbon atoms (C₁-C₈ alkyl) - should be noted. Examples of alkyl groups include, but are not limited to: methyl; ethyl; propyl; isopropyl; n-butyl; isobutyl; sec-butyl; tert-butyl; n-pentyl; n-hexyl; n-heptyl; and, 2-ethylhexyl. In the present invention, such alkyl groups may be unsubstituted or may be substituted with one or more halogen. Where applicable for a given moiety (R), a tolerance for one or more non-halogen substituents within an alkyl group will be noted in the specification.

The term *"C₁-C₁₈ hydroxyalkyl"* as used herein refers to a HO-(alkyl) group having from 1 to 18 carbon atoms, where the point of attachment of the substituent is through the oxygen-atom and the alkyl group is as defined above.

An *"alkoxy group"* refers to a monovalent group represented by -OA where A is an alkyl group: nonlimiting examples thereof are a methoxy group, an ethoxy group and an iso-propyloxy group. The term *"C₁-C₁₈ alkoxyalkyl"* as used herein refers to an alkyl group having an alkoxy substituent as defined above and wherein the moiety (alkyl-O-alkyl) comprises in total from 1 to 18 carbon atoms: such groups include methoxymethyl (-CH₂OCH₃), 2-methoxyethyl (-CH₂CH₂OCH₃) and 2-ethoxyethyl. Analogously, the term "C₇-C₁₈ alkoxyaryl" as used herein refers to an aryl group having an alkoxy substituent as defined above and wherein the moiety (aryl-O-alkyl) has in total from 7 to 18 carbon atoms.

The term *"C₂-C₄ alkylene"* as used herein, is defined as saturated, divalent hydrocarbon radical having from 2 to 4 carbon atoms. In general in the present disclosure, such alkylene groups may be unsubstituted or may be substituted with one or more halogen.

The term *"C₃ -C₁₈ cycloalkyl"* is understood to mean a saturated, mono- or polycyclic hydrocarbon group having from 3 to 18 carbon atoms. In the present invention, such cycloalkyl groups may be unsubstituted or may be substituted with one or more halogen. Where applicable for a given moiety (R), a tolerance for one or more non-halogen substituents within a cycloalkyl group will be noted in the specification. Examples of cycloalkyl groups include: cyclopropyl; cyclobutyl; cyclopentyl; cyclohexyl; cycloheptyl; cyclooctyl; adamantane; and, norbornane.

As used herein, *"C₃-C₁₈ cycloalkylene"* means a divalent radical formed by the removal of two hydrogen atoms from one or more rings of a cycloalkyl group having from 3 to 18 carbon atoms. In general in the present disclosure, such cycloalkylene groups may be unsubstituted or may be substituted with one or more halogen.

As used herein, an *"C₆-C₁₈ aryl"* group used alone or as part of a larger moiety - as in *"aralkyl group"* - refers to monocyclic, bicyclic and tricyclic ring systems in which the monocyclic ring system is aromatic or at least one of the rings in a bicyclic or tricyclic ring system is aromatic. The bicyclic and tricyclic ring systems include benzofused 2-3 membered carbocyclic rings. In the present invention, such aryl groups may be unsubstituted or may be substituted with one or more halogen. Where applicable for a given moiety (R), a tolerance for one or more non-halogen substituents within an aryl group will be noted in the specification. Exemplary aryl groups include: phenyl; (C1-C4)alkylphenyl, such as tolyl and ethylphenyl; indenyl; naphthalenyl, tetrahydronaphthyl, tetrahydroindenyl; tetrahydroanthracenyl; and, anthracenyl. And a preference for phenyl groups may be noted.

As used herein, *"C₆-C₁₈ arylene"* means a divalent radical formed by the removal of two hydrogen atoms from one or more rings of a cycloalkyl group having from 3 to 18 carbon atoms. In general in the present disclosure, such arylene groups may be unsubstituted or may be substituted with one or more halogen.

As used herein, *"C₂-C₂₀ alkenyl"* refers to hydrocarbyl groups having from 2 to 20 carbon atoms and at least one unit of ethylenic unsaturation. The alkenyl group can be straight chained, branched or cyclic and may optionally be substituted with one or more halogen. Where applicable for a given moiety (R), a tolerance for one or more non-halogen substituents within an alkenyl group will be noted in the specification. The term *"alkenyl"* also encompasses radicals having "cis" and "trans" configurations, or alternatively, "*E*" and "*Z*" configurations, as appreciated by those of ordinary skill in the art. In general, however, a preference for unsubstituted alkenyl groups containing from 2 to 10 (C₂₋₁₀) or 2 to 8 (C₂₋₈) carbon atoms should be noted. Examples of said C₂-C₁₂ alkenyl groups include, but are not limited to: -CH=CH₂; -CH=CHCH₃; -CH₂CH=CH₂; - C(=CH₂)(CH₃); -CH=CHCH₂CH₃; -CH₂CH=CHCH₃; -CH₂CH₂CH=CH₂; -CH=C(CH₃)₂; - CH₂C(=CH₂)(CH₃); -C(=CH₂)CH₂CH₃; -C(CH₃)=CHCH₃; -C(CH₃)CH=CH₂; - CH=CHCH₂CH₂CH₃; -CH₂CH=CHCH₂CH₃; -CH₂CH₂CH=CHCH₃; -CH₂CH₂CH₂CH=CH₂; - C(=CH₂)CH₂CH₂CH₃; -C(CH₃)=CHCH₂CH₃; -CH(CH₃)CH=CHCH; -CH(CH₃)CH₂CH=CH₂; - CH₂CH=C(CH₃)₂; 1-cyclopent-1-enyl; 1-cyclopent-2-enyl; 1-cyclopent-3-enyl; 1-cyclohex-1-enyl; 1-cyclohex-2-enyl; and, 1-cyclohexyl-3-enyl.

As used herein, *"alkylaryl"* refers to alkyl-substituted aryl groups, both groups being defined as above. Further, as used herein "aralkyl" means an alkyl group substituted with an aryl radical as defined above.

The term *"hetero"* as used herein refers to groups or moieties containing one or more heteroatoms, such as N, O, Si and S. Thus, for example *"heterocyclic"* refers to cyclic groups having, for example, N, O, Si or S as part of the ring structure. *"Heteroalkyl", "heterocycloalkyl"* and *"heteroaryl"* moieties are alkyl, cycloalkyl and aryl groups as defined hereinabove, respectively, containing N, O, Si or S as part of their structure.

The present materials and compositions may be defined herein as being *"substantially free"* of certain compounds, elements, ions or other like components. The term *"substantially free"* is intended to mean that the compound, element, ion or other like component is not deliberately added to the material or composition and is present, at most, in only trace amounts which will have no (adverse) effect on the desired properties of the material or composition. An exemplary trace amount is less than 1000 ppm by weight of the material or composition. The term *"substantially free"* encompasses those embodiments where the specified compound, element, ion, or other like component is completely absent from the material or composition or is not present in any amount measurable by techniques generally used in the art.

The term *"anhydrous"* as used herein has equivalence to the term *"substantially free of water".* Water is not deliberately added to a given composition and is present, at most, in only trace amounts which will have no (adverse) effect on the desired properties of the composition.

### DETAILED DESCRIPTION OF THE INVENTION

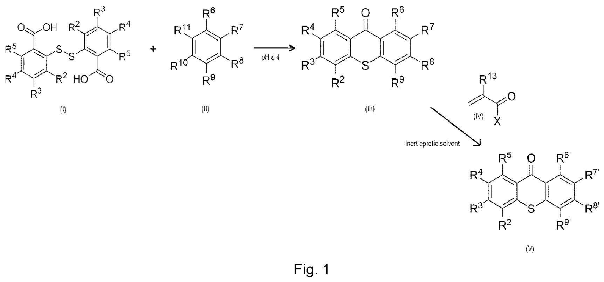

The two steps of the method of the present disclosure are illustrated in Fig. 1 appended hereto.

### Step i)

The first stage of the present disclosure includes the provision, as a reactant of a disulfide compound in accordance with Formula (I) herein below: 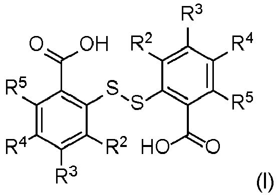 wherein: R² to R⁵ are independently selected from H or C₁-C₆ alkyl.

It is preferable that R² to R⁵ be independently selected from H or C₁-C₄ alkyl and more preferable that R² to R⁵ be independently selected from H or C₁-C₂ alkyl. In an important embodiment, R² to R⁵ are each H, whereby the reactant in accordance with Formula (I) is 2,2'-dithiodibenzoic acid.

The first stage of the present disclosure also encompasses the provision of a hydroxyl functional reactant in accordance with Formula (II) herein below: 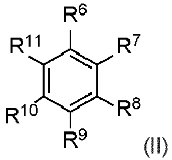 wherein:
R⁶ to R⁹ are independently selected from H, C₁-C₆ alkyl, C₁-C₆ alkoxy, C₁-C₁₂ alkoxyalkyl, SR¹², COOR¹² and N(R¹²)₂;
R¹⁰ and R¹¹ are H; and,
each R¹² is independently selected from C₁-C₆ alkyl or C₆-C₁₈ aryl,
subject to the proviso that n of radicals R⁶ to R⁹ are R^{b}(OH), wherein n is an integer of from 1 to 3 and for each of said n radicals R^{b} is independently selected from a covalent bond, C₂-C₁₂ alkylene, C₃-C₁₈ cycloalkylene or C₆-C₁₈ arylene.

As regards Formula (II), it is preferred that:
R⁶ to R⁹ are independently selected from the H, C₁-C₄ alkyl, C₁-C₄ alkoxy, C₁-C₈ alkoxyalkyl, COOR¹² and N(R¹²)₂;
R¹⁰ and R¹¹ are H; and,
each R¹² is independently selected from C₁-C₄ alkyl or C₆-C₁₈ aryl,
subject to the proviso that n of radicals R⁶ to R⁹ are R^{b}OH, wherein n is an integer of 1 or 2.

In this first stage (i)), the disulfide compound of Formula (I) is reacted with the hydroxyl functional compound of Formula (II) to yield a hydroxyl functional compound in accordance with Formula (III).

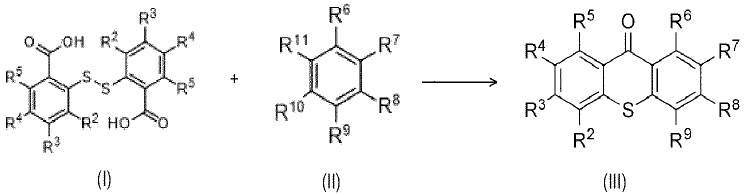

For completeness, within the compound of Formula (III) depicted above and in Figure 1, substituents R² to R⁹ have the meanings accorded above.

The reaction of this step is performed in an acidic medium having a pH at or below 4.0. The acidic medium may, in certain embodiments, have a pH at or below 3.5 or at or below 3.0. Illustrative acidic media which may be used include: concentrated sulphuric acid; concentrated mixtures of sulphuric acid and acetic acid; concentrated hydrochloric acid; concentrated phosphoric acid (H₃PO₄); acetic acid; and, mixtures of acetic acid and acetic anhydride. In forming the acidic reaction medium, the number of moles of acid should typically be at least ten times that of the reactant compound (I).

The reaction temperature should be below the boiling point of the acidic medium and is typically selected to be in the range from 20°C to 120°C, for example from 40 to 120°C or from 60 to 100°C. In certain embodiments, the reaction may be performed under a multi-stage regime with respect to temperature. For instance, the reactants and acid of the medium may be initially mixed at room temperature and the temperature of the mixture - under continual stirring - then slowly elevated to a range of from 40 to 120°C for a first duration, for instance of from 1 to 5 hours. Under maintained stirring, the temperature of the mixture may then be allowed to return to room temperature and the reaction permitted to continue for a second duration, for instance from 2 to 12 hours.

Subject to the proviso that the acidic medium should not be boiling, the process pressure is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The progress of the above reaction step i) may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, the compound of Formula (III) is precipitated from the product mixture, typically by introducing that mixture into boiling water. The precipitate may then be separated and washed, typically with water.

The crude solid product obtained may subsequently be purified by methods known in the art, including but not limited to solvent extraction, filtration, evaporation, distillation, (re-)crystallization and chromatography. Mention may in particular be made of the re-crystallization of the product from a C₁-C₄ alkanol, such as methanol.

### Step ii)

In this step, the compound of Formula (III) is reacted, in an inert aprotic solvent, with a (meth)acrylating agent selected from the group consisting of (meth)acryloyl halides and (meth)acrylic anhydrides. More particularly, said methacrylating agent has the Formula (IV) below: 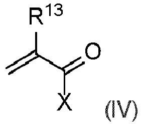 wherein: X is halide or -OC(O)C(R¹³)=CH₂ ; and,
R¹³ is H or C₁ alkyl.

In a preferred embodiment X is halide. Exemplary (meth)acryloyl halides include: acryloyl fluoride; acryloyl chloride; acryloyl bromide; acryloyl iodide; methacryloyl chloride; and, methacroyloyl bromide. The use of (meth)acryloyl chloride as the (meth)acrylating agent may be mentioned in particular.

The reaction of this step ii) is depicted below:

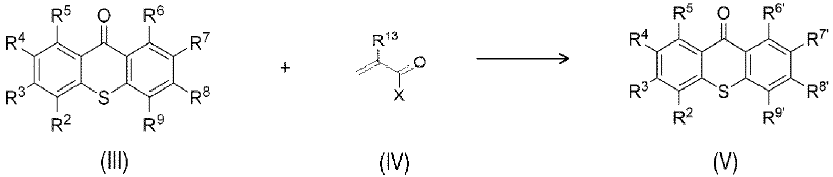

For completeness, within the compound of Formula (V) as depicted, substituents R² to R⁵ have the meanings accorded above. R^{6'} to R^{9'} correspond to R⁶ to R⁹ save for the conversion of the n hydroxyl groups to (meth)acrylate groups. Thereby in Formula (V):
R^{6'} to R^{9'} are independently selected from H, C₁-C₆ alkyl, C₁-C₆ alkoxy, C₁-C₁₂ alkoxyalkyl, SR¹², COOR¹² and N(R¹²)₂; and,
each R¹² is independently selected from C₁-C₆ alkyl or C₆-C₁₈ aryl,
subject to the proviso that n of radicals R^{6'} to R^{9'} are independently selected from -R^{b}OC(O)C(R¹³)=CH₂ wherein:
   R¹³ is H or C₁ alkyl;
   n is an integer of from 1 to 3, preferably 1 or 2; and,
   for each of said n radicals, R^{b} is independently selected from a covalent bond, C₂-C₁₂ alkylene, C₃-C₁₈ cycloalkylene or C₆-C₁₈ arylene.

The (meth)acrylating agent should be employed in an amount such that the number of moles of (meth)acrylate groups provided by said (meth)acrylating agent is at least equimolar with the number of moles of moles of hydroxyl groups provided by the compound of Formula (III). In some embodiments, it is preferred that the molar ratio of (meth)acrylate groups provided by said (meth)acrylating agent to hydroxyl groups provided by the compound of Formula (III) is from 1:1 to 2:1, for example from 1.1:1 to 1.5:1 or from 1.1:1 to 1.4: 1. The provision of a stoichiometric excess of the (meth)acrylate groups can promote the complete reaction of the hydroxyl groups and can compensate for the reaction of said (meth)acrylate groups with any water entrained within the inert aprotic solvent.

Where the (meth)acrylating agent is a (meth)acryloyl halide, the reaction is desirably performed in the presence of a base which acts as a hydrogen-halide trap. The base would conventionally be employed in a supra-stoichiometric amount relative to the reactant (meth)acryloyl halide. Exemplary bases include but are not limited to: alkali metal hydroxides; alkali metal alkoxides, in particular C₁-C₄ aliphatic alkoxides of lithium sodium or potassium; alkaline earth metal hydroxides; and, tertiary amines.

The use of one or more tertiary amines is preferred in this regard. Without intention to limit the present disclosure, the utilized tertiary amines should be liquid under the reaction conditions. Alternatively or additionally, it is preferred for the or each tertiary amine used to be selected from tri(C₁-C₁₂)alkyl amines, di(C₁-C₁₂)alkyl(C₃-C₈)cycloalkyl amines or tri(C₁-C₁₀)alkenyl amines. A particular preference for the use of tri(C₁-C₁₂)alkyl amines may be mentioned, of which examples include but are not limited: trimethylamine; ethyldimethylamine; diethylmethylamine; triethylamine; triisopropylamine; tri-n-propylamine; tri-n-butylamine; tri-sec-butylamine; dibutylpentylamine; n-butyl-octyl-sec-butylamine; tripentylamine; and, trihexylamine.

This step is still further performed in the presence of inert aprotic solvent. Examples of suitable aprotic solvents, which may be used alone or in combination, include but are not limited to: pentane; hexane; heptanes; cyclopentane; cyclohexane; cycloheptane; dimethylether; chloroform; dimethyl carbonate; ethylmethyl carbonate; diethyl carbonate; toluene; o-xylene; m-xylene; p-xylene; ethylbenzene; 2-propylbenzene (cumene); 2-isopropyltoluene (*o-cymol*); 3-isopropyltoluene (*m-cymol*); 4-isopropyltoluene (*p-cymo*/); 1,3,5-trimethylbenzene (*mesitylene*); acetonitrile; N,N-di(C₁-C₄)alkylacylamides, such as N,N-dimethylformamide (DMF) and N,N-dimethylacetamide (DMAc); hexamethylphosphoramide; N-methylpyrrolidone; pyridine; esters, such as (C₁-C₈)alkyl acetates, ethoxydiglycol acetate, dimethyl glutarate, dimethyl maleate, dipropyl oxalate, ethyl lactate, benzyl benzoate, butyloctyl benzoate and ethylhexyl benzoate; ketones, such as acetone, ethyl ketone, methyl ethyl ketone (*2-butanone*) and methyl isobutyl ketone; ethers, such as tetrahydrofuran (THF), 2-methyltetrahydrofuran (2-MeTHF) and 1,2-dimethoxyethane; 1,3-dioxolane; dimethylsulfoxide (DMSO); and, dichloromethane (DCM).

Whilst it is not critical, it is preferred that the reaction of this step be performed under anhydrous conditions. Where necessary, exposure to atmospheric moisture may be avoided by providing the reaction vessel with an inert, dry gaseous blanket. Whilst dry nitrogen, helium and argon may be used as blanket gases, precaution should be used when common nitrogen gases are used as a blanket, because such nitrogen may not be dry enough on account of its susceptibility to moisture entrainment; the nitrogen may require an additional drying step before use herein.

The process pressure is not critical: as such, the reaction can be run at sub-atmospheric, atmospheric, or super-atmospheric pressures but pressures at or slightly above atmospheric pressure are preferred. Mention in this regard may be made of pressures of from 50 to 200 kPa, for example from 100 to 200 kPa.

The reaction temperature is typically from -40 to 20°C, for example from -20 to 20°C. As the reaction is generally exothermic, some cooling might be required as it progresses.

In certain embodiments, this stage of the reaction may be carried out in the presence of a polymerization inhibitor or a polymerization retarder. Polymerization inhibitors inhibit polymerization reactions from occurring and exemplary compounds which may have utility as inhibitors herein include: N,N'-dialkylphenylenediamines; N,N'-diarylphenylenediamines; N-aryl-N'-alkylphenylenediamines; and, quinone diimides. Polymerization retarders, while they slow down the rate of polymerization reactions, are not as effective as polymerization inhibitors in this context but, conversely, are consumed less rapidly. Exemplary polymerization retarders include quinone methide compounds, as disclosed in *inter alia* US Patent No. 4,003,800, US Patent No. 5,583,247 and US Patent No. 7,045,647. Combinations of a polymerization inhibitor and polymerization retarder are disclosed in US 20200017610 A1 (Masere et al.).

The progress of the reaction of step ii) may be monitored by known techniques of which mention may be made of ¹H NMR, Fourier Transform Infrared Spectroscopy, Ultra Performance Liquid Chromatography (UPLC) or thin layer chromatography (TLC). Upon completion of the reaction, the reaction mixture is washed with water and the organic product phase separated from the aqueous phase. The aprotic solvent may then be wholly or partially removed from that organic phase: this should effected under reduced pressure and without elevating the temperature of the solution significantly above room temperature and, preferably, without exceeding 40°C or even 35°C. The crude solid product may thus either be obtained following complete removal of the solvent or may be formed by subsequent cooling of a concentrated organic phase.

The crude solid product obtained may subsequently be purified by methods known in the art, including but not limited to solvent extraction, filtration, evaporation, distillation, (re-)crystallization and chromatography.

### Illustrative Embodiment of the Method

In accordance with an illustrative embodiment of the present disclosure, there is provided a method of preparing a compound of Formula (VA): 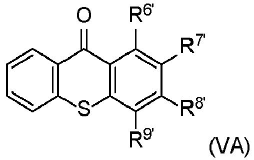 wherein: R^{6'} to R^{9'} are independently selected from H, C₁-C₄ alkyl, C₁-C₄ alkoxy, C₁-C₈ alkoxyalkyl, COOR¹² and N(R¹²)₂; and,
each R¹² is independently selected from C₁-C₄ alkyl or C₆-C₁₈ aryl,
subject to the proviso that n of radicals R^{6'} to R^{9'} are -OC(O)C(R¹³)=CH₂ wherein R¹³ is H or C₁ alkyl and further wherein n is an integer of 1 or 2,
said method comprising the steps of:
i) reacting a disulfide compound of Formula (IA) with a hydroxyl functional compound of Formula (II) to yield a compound of Formula (IIIA) 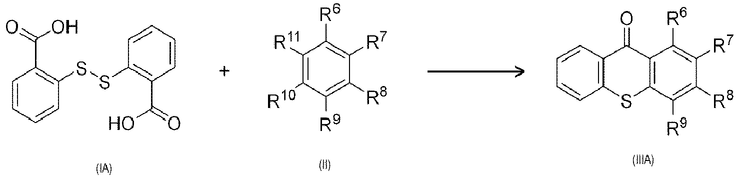 wherein:
   said reaction is performed in an acidic medium having a pH at or below 3.0;
   said reaction is performed at a temperature below the boiling point of the acidic medium and in the range from 40 to 120°C;
   R⁶ to R⁹ correspond to said substituents R^{6'} to R^{9'} and are independently selected from the H, C₁-C₄ alkyl, C₁-C₄ alkoxy, C₁-C₈ alkoxyalkyl, COOR¹² and N(R¹²)₂;
   R¹⁰ and R¹¹ are H; and,
   each R¹² is independently selected from C₁-C₄ alkyl or C₆-C₁₈ aryl,
   subject to the proviso that n of radicals R⁶ to R⁹ are -OH, wherein n is an integer of 1 or 2;
ii) reacting in an inert aprotic solvent said compound of Formula (IIIA) with a compound of Formula (IV) to yield said compound of Formula (VA) 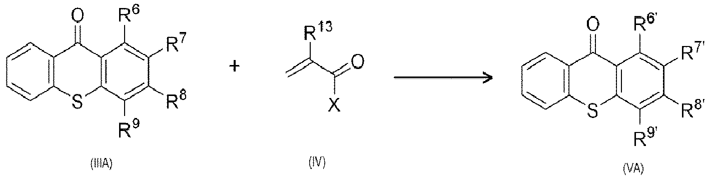 wherein: X is chloride;
   the molar ratio of (meth)acrylate groups provided by said compound of Formula (IV) to hydroxyl groups provided by said compound of Formula (IIIA) is from 1.1:1 to 1.5:1; and,
   the reaction of step ii) is performed in the presence of a base selected from the group consisting of: tri(C₁-C₁₂)alkyl amines; di(C₁-C₁₂)alkyl(C₃-C₈)cycloalkyl amines; tri(C₁-C₁₀)alkenyl amines; and, mixtures thereof.

### Formation of the copolymers by Free Radical Polymerization

The present disclosure also provides for a polymer obtained by free radical polymerization of the compound of Formula (V) of which the compound of Formula (VA) is one example. In particular, the present disclosure provides a copolymer obtained by free radical polymerization, said copolymer comprising, based on the total weight of monomers: from 0.1 to 10 wt.% of a) at least one compound in accordance with Formula (V); and, from 90 to 99.9 wt.% of b) at least one ethylenically unsaturated non-ionic monomer which does not bear an epoxide group or a moiety decomposable under photo-irradiation to form a radical.

The copolymers of the present disclosure are prepared by free radical polymerization. As would be recognized by the skilled practitioner, free radical polymerization is constituted by three stages: initiation, wherein the decomposition of an initiator generates active free radicals which, possessing unpaired electrons, react with the monomers present to generating an initiating radicals chain; propagation, wherein the formed initiating radicals chain attacks a second monomer molecule transferring its active center to the attacked molecule, which process is repeated, growing the polymer chain; and, termination, wherein the growth of macromolecular chains stops, and the polymerization terminates by disabling the active center. The two most common termination mechanisms in radical polymerizations are combination and deprotonation.

Free radical polymerization may be performed in bulk, in emulsion, in suspension or in solution. Without specific intention to limit the present disclosure, the copolymers are preferably prepared by free radical solution polymerization: by this is meant that a solution of the monomers in a solvent, which is also capable of dissolving the copolymer is polymerized by a free radical polymerization, that is in the presence of the polymerization initiator. The concentration of the monomers in the solution may vary but it will be typical for the ratio by weight of monomer to solvent to be in the range from 1:20 to 2:1, for example from 1:2 to 1.5:1.

The free radical solution polymerization reaction should desirably be carried out in the presence of a polar solvent having a boiling point of at least 20°C, for instance at least 30°C or at least 40°C, as measured at 1 atmosphere pressure (1.01325 Bar). Examples of such polar solvents, which may be used alone or in combination, include but are not limited to: water; C₁-C₈ alkanols such as methanol, ethanol, n-propanol, isopropanol, n-butanol, sec-butanol and isobutanol; acetonitrile; N,N-di(C₁-C₄)alkylacylamides, such as N,N-dimethylformamide (DMF) and N,N-dimethylacetamide (DMAc); hexamethylphosphoramide; N-methylpyrrolidone; pyridine; esters, such as (C₁-C₈)alkyl acetates, ethoxydiglycol acetate, dimethyl glutarate, dimethyl maleate, dipropyl oxalate, ethyl lactate, benzyl benzoate, butyloctyl benzoate and ethylhexyl benzoate; ketones, such as acetone, ethyl ketone, methyl ethyl ketone (*2-butanone*) and methyl isobutyl ketone; ethers, such as tetrahydrofuran (THF), 2-methyltetrahydrofuran (2-MeTHF) and 1,2-dimethoxyethane; 1,3-dioxolane; dimethylsulfoxide (DMSO); and, dichloromethane (DCM). In an exemplary embodiment, the polymerization reaction is performed in the presence of a (C₁-C₈)alkyl acetate and, in particular ethyl acetate.

As noted above, the free radical polymerization will be triggered by means of at least one radical generating thermal initiator. As will be understood by a person skilled in the art, a thermal initiator is a compound which can be activated by thermal energy to generate a radical thereof upon, for instance, heating or irradiation of the infrared or microwave wavelength regions. The polymerization composition should conventionally comprise from 0.1 to 1 wt.%, for example from 0.1 to 0.5 wt.% of said at least one radical generating thermal initiator, based on the total weight of the polymerizable monomers.

Without intention to limit the present invention, an exemplary class of radical generating thermal initiators suitable for use herein are organic peroxides, selected for example from: cyclic peroxides; diacyl peroxides; dialkyl peroxides; hydroperoxides; peroxycarbonates; peroxydicarbonates; peroxyesters; and, peroxyketals.

While certain peroxides - such as dialkyl peroxides - have been disclosed as useful initiators in *inter alia* US Patent No. 3,419,512 (Lees) and US Patent No. 3,479,246 (Stapleton) and indeed may have utility herein, hydroperoxides represent a preferred class of initiator for the present invention. Further, whilst hydrogen peroxide itself may be used, the most desirable polymerization initiators are the organic hydroperoxides. For completeness, included within the definition of hydroperoxides are materials such as organic peroxides or organic peresters which decompose or hydrolyze to form organic hydroperoxides *in situ*: examples of such peroxides and peresters are cyclohexyl and hydroxycyclohexyl peroxide and t-butyl perbenzoate, respectively.

In an embodiment of the invention, the radical generating thermal initiator comprises or consists of at least one hydroperoxide compound represented by the formula:

RPOOH

wherein: RP is an aliphatic or aromatic group containing up to 18 carbon atoms, and preferably wherein: R^{p} is a C₁-C₁₂ alkyl, C₆-C₁₈ aryl or C₇-C₁₈ aralkyl group.

As exemplary peroxide initiators, which may be used alone or in combination, there may be mentioned: cumene hydroperoxide (CHP); para-menthane hydroperoxide; t-butyl hydroperoxide (TBH); t-butyl perbenzoate; t-butyl peroxy pivalate; di-t-butyl peroxide; t-butyl peroxy acetate; t-butyl peroxy-2-hexanoate; t-amyl hydroperoxide; 1,2,3,4-tetramethylbutyl hydroperoxide; benzoyl peroxide; dibenzoyl peroxide; 1,3-bis(t-butylperoxyisopropyl) benzene; diacetyl peroxide; butyl 4,4-bis (t-butylperoxy) valerate; p-chlorobenzoyl peroxide; t-butyl cumyl peroxide; di-t-butyl peroxide; dicumyl peroxide; 2,5-dimethyl-2,5-di-t-butylperoxyhexane; 2,5-dimethyl-2,5-di-t-butyl-peroxyhex-3-yne; and, 4-methyl-2,2-di-t-butylperoxypentane.

Without intention to limit the present invention, a further exemplary class of radical generating thermal initiators suitable for use herein are azo polymerization initiators, selected for example from: azo nitriles; azo esters; azo amides; azo amidines; azo imidazoline; and, macro azo initiators.

As representative examples of suitable azo polymerization initiators may be mentioned: 2,2'-azobis (2-methylbutyronitrile); 2,2'-azobis(isobutyronitrile); 2,2'-azobis(2,4-dimethylvaleronitrile); 2,2'-azobis(4-methoxy-2,4-dimethylvaleronitrile); 1,1'-azobis(cyclohexane-1-carbonitrile); 4,4'-azobis(4-cyanovaleric acid); dimethyl 2,2'-azobis(2-methylpropionate); 2,2'-azobis[2-methyl-N-(2-hydroxyethyl)propionamide]; 2,2'-azobis (N-butyl-2-methylpropionamide); 2,2'-azobis[2-(2-imidazolin-2-yl)propane]dihydrochloride; 2,2'-azobis[2-(2-imidazolin-2-yl)propane]; 2,2'-azobis(2-methylpropionamidine)dihydrochloride; 2,2'-azobis[N-(2-carboxyethyl)-2-methylpropionamidine]tetrahydrate; 4,4-azobis(4-cyanovaleric acid), polymer with alpha, omega-bis(3-aminopropyl)polydimethylsiloxane (VPS-1001, available from Wako Pure Chemical Industries, Ltd.); and, 4,4'-azobis(4-cyanopentanoicacic)·polyethyleneglycol polymer (VPE-0201, available from Wako Pure Chemical Industries, Ltd.).

Redox initiators are a combination of an oxidizing agent and a reducing agent and may also have utility in the present invention. Suitable oxidizing agents may be selected from the group consisting of cyclic peroxides, diacyl peroxides, dialkyl peroxides, hydroperoxides, peroxycarbonates, peroxydicarbonates, peroxyesters and peroxyketals. The corresponding reducing agent may be selected from the group consisting of: alkali metal sulfites; alkali metal hydrogensulfites; alkali metal metabisulfites; formaldehyde sulfoxylates; alkali metal salts of aliphatic sulfinic acids; alkali metal hydrogensulfides; salts of polyvalent metals, in particular Co(ll) salts and Fe(ll) salts such iron(II) sulfate, iron(II) ammonium sulfate or iron(II) phosphate; dihydroxymaleic acid; benzoin; ascorbic acid; and, reducing saccharides, such as sorbose, glucose, fructose and/or dihydroxyacetone.

Aside from initiators, it is considered that the free radical polymerization may be conducted in the presence of chain transfer agents which act to transfer free radicals and which reduce the molecular weight of the obtained polymer and / or control chain growth in the polymerization. When added, the chain transfer agent should constitute from 0.01 to 1 wt.%, based on the total weight of polymerizable monomers.

The process for producing the copolymer is preferably carried out in a manner that the copolymer has a number average molecular weight (Mn) of from 50000 to 500000 daltons, for example from 75000 to 300000 daltons or from 100000 to 250000 daltons. The amount of polymerization initiator and any chain transfer agents present will be determinative of the number average molecular weight of the copolymer, although the choice of solvent may also be important.

Without intention to limit the present invention, conventional polymerization conditions will include a temperature in the range of from 0 to 175°C, for example from 25 to 125°C or from 50 to 100°C. The polymerization pressure is generally not critical and, as such, the polymerization may be conducted at sub-atmospheric, atmospheric or super-atmospheric pressure. Pressure aside, the polymerization may be conducted, where necessary, under the exclusion of oxygen: the reaction vessel may be provided with an inert, dry gaseous blanket of, for example, nitrogen, helium and argon.

For completeness, it is considered that the present polymerization may be performed as a batch or semi-batch procedure or as a continuous procedure. As would be recognized by the skilled artisan, in the batch procedure, the monomers to be polymerized and optionally the solvent used in the polymerization procedure are charged to a reaction vessel, while the majority or the total amount of the polymerization initiator is added to the reaction vessel over the course of the polymerization. In a semi-batch procedure, at least a portion - up to and including the total amount - of the polymerization initiator and solvent are initially charged to the reaction vessel: a small portion of the monomers may also be so-charged but the majority of monomers to be polymerized are added to the reaction vessel over the course of the polymerization. In a continuous process the monomers, polymerization initiator and, optionally, the solvent are continuously added to a reaction vessel and the obtained polymer is continuously discharged from the polymerization vessel.

A preference may be mentioned for the performance of the present polymerization as a semi-batch procedure. In particular, at least 75 wt.% of the total weight of monomers to be polymerized should be added to the reaction vessel over the course of the polymerization reaction.

There is no particular intention to limit the timing at which the different functional monomers - part a) and part b) - of the copolymer are introduced into the polymerization procedure. The monomers may be provided to the polymerization vessel at a fixed molar ratio either at the start of the polymerization (for a batch process) or through the entire polymerization process (for semi-batch and continuous processes). In the alternative, the molar ratio of the monomers types may be varied during the course of the polymerization: this is intended to encompass that embodiment where, during the course of the polymerization, only the monomers of part a) are added to the polymerization vessel or conversely only monomers of part b) are added to the polymerization vessel. The skilled artisan may make determinations of suitable molar ratios based on the desired form or randomness of the copolymer and the reactivity ratios of the monomers.

The copolymer reaction product may be isolated and purified using methods known in the art, of which mention may be made of extraction, evaporation, crystallization, distillation and chromatography as suitable techniques. Where a free radical solution polymerization is performed, it is most convenient that the copolymer be isolated by distilling off the solvent and any unreacted starting materials under reduced pressure. Where it is intended that the (optionally purified) copolymer be stored upon production, the polymers should be disposed in a vessel with an airtight and moisture-tight seal. The storage vessel should not permit the penetration of photo-irradiation.

The following examples are illustrative of the present invention and are not intended to limit the scope of the invention in any way.

### EXAMPLES

The following commercial compounds are used in the Example:

### Example 1: Synthesis of 2-hydroxy-3-(propan-2-yl)-9H-thioxanthen-9-one

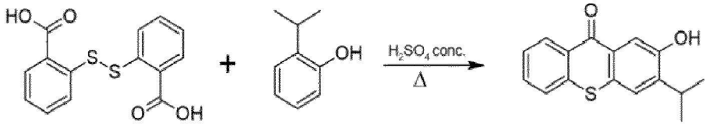

5.0g (16.33 mmol) of 2,2'-dithiobenzoic acid was suspended in 50 ml of concentrated (95%) sulfuric acid. 13g (95.5 mmol) of 2-(propan-2-yl)phenol was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; ca. 50% yield.

### Example 2: Synthesis of 9-oxo-3-(propan-2-yl)-9H-thioxanthen-2-yl prop-2-enoate

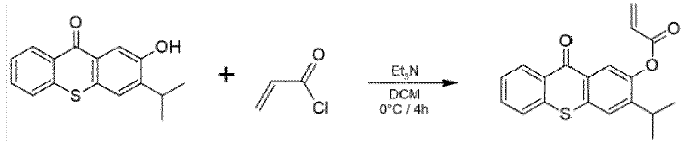

5g (18.5 mmol) of 2-hydroxy-3-(propan-2-yl)-9H-thioxanthen-9-one was dissolved in 200ml of dry dichloromethane. After the addition of 4g (39.5 mmol) of triethylamine, the reaction mixture was cooled to 0°C under stirring and a nitrogen atmosphere. 2ml (24.5 mmol) of acryloyl chloride was added dropwise through a septum. After stirring for 4 hours at 0°C, the reaction mixture was further stirred overnight at room temperature. The obtained mixture was carefully washed twice with 50 ml of deionized water. The organic phase was concentrated on a rotavapor to a volume of approximately 15 ml whilst not exceeding 40°C. After cooling to 0°C, the precipitate was filtered off and washed with methanol. The filtered product was recrystallized from methanol and dried under vacuum. *Product:* yellow powder; ca. 50% yield.

### Example 3: Synthesis of 2-hydroxy-1,3-dimethyl-9H- thioxanthen-9-one

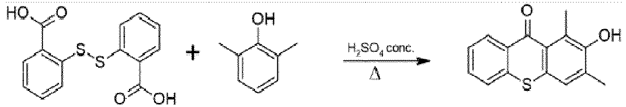

5.0g (16.33 mmol) of 2,2'-dithiobenzoic acid was suspended in 50 ml of concentrated (95%) sulfuric acid. 95.5 mmol of 2,6-dimethylphenol was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; ca. 50% yield.

The obtained 2-hydroxy-1,3-dimethyl-9H- thioxanthen-9-one was reacted with acryloyl chloride in accordance with the procedure of Example 2.

### Example 4: Synthesis of 1-hydroxy-4-(2-methoxyethyl)-9H-thioxanthen-9-one

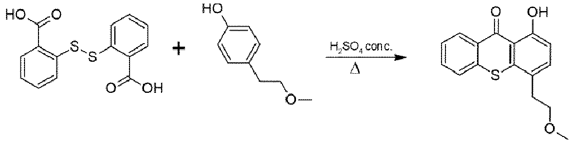

5.0g (16.33 mmol) of 2,2'-dithiobenzoic acid was suspended in 50 ml of concentrated (95%) sulfuric acid. 95.5 mmol of 4-(2-methoxyethyl)phenol was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; ca. 50% yield.

The obtained 1-hydroxy-4-(2-methoxyethyl)-9H-thioxanthen-9-one was reacted with acryloyl chloride in accordance with the procedure of Example 2.

### Example 5: 3-acetyl-2-hydroxy-9H-thioxanthen-9-one

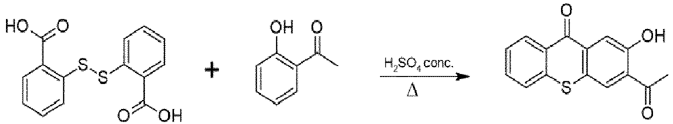

5.0g (16.33 mmol) of 2,2'-dithiobenzoic acid was suspended in 50 ml of concentrated (95%) sulfuric acid. 95.5 mmol of 1-(2-hydroxyphenyl)ethan-1-one was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; ca. 50% yield.

The obtained 3-acetyl-2-hydroxy-9H-thioxanthen-9-one was reacted with acryloyl chloride in accordance with the procedure of Example 2.

### Example 6: Synthesis of 3-(dimethylamino)-1-hydroxy-9H-thioxanthen-9-one

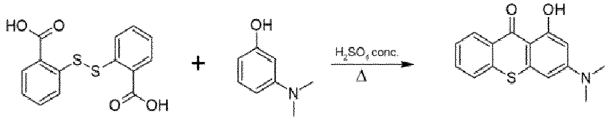

5.0g (16.33 mmol) of 2,2'-dithiobenzoic acid was suspended in 50 ml of concentrated (95%) sulfuric acid. 95.5 mmol of 3-(dimethylamino)phenol was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; ca. 50% yield.

The obtained 3-(dimethylamino)-1-hydroxy-9H-thioxanthen-9-one was reacted with acryloyl chloride in accordance with the procedure of Example 2.

### Example 7: 2-hydroxy-9-oxo-9H-thioxanthene-3-carboxylate

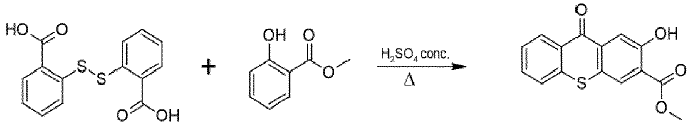

5.0g (16.33 mmol) of 2,2'-dithiobenzoic acid was suspended in 50 ml of concentrated (95%) sulfuric acid. 95.5 mmol of 2-hydroxybenzoate was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; c. 50% yield.

The obtained 2-hydroxy-9-oxo-9H-thioxanthene-3-carboxylate was reacted with acryloyl chloride in accordance with the procedure of Example 2.

### Example 8: Synthesis of 3-ethoxy-2-hydroxy-9H-thioxanthen-9-one

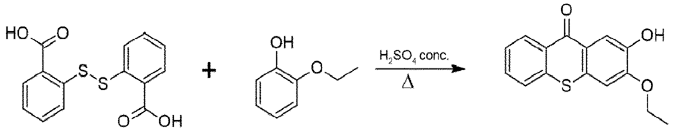

5.0g (16.33 mmol) of 2,2'-dithiobenzoic acid was suspended in 50 ml of concentrated (95%) sulfuric acid. 95.5 mmol of 2-ethoxyphenol was added to the suspension over 10 minutes: within this time, the mixture was heated to approximately 50°C. The reaction mixture was then heated to 80°C, which temperature was maintained for three hours. After that time, the mixture was cooled to room temperature and stirred overnight. The obtained mixture was added dropwise into 500 ml of boiling, deionized water. The resultant precipitate was filtered off and washed once with 50 ml of boiling water and once with 100 ml of cold water. The resulting product was dried under vacuum. *Product:* green powder; c. 50% yield.

The obtained 3-ethoxy-2-hydroxy-9H-thioxanthen-9-one was reacted with acryloyl chloride in accordance with the procedure of Example 2.

In view of the foregoing description and example, it will be apparent to those skilled in the art that equivalent modifications thereof can be made without departing from the scope of the claims.

## Claims

1. A method of preparing a compound of Formula (V): 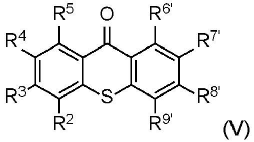 wherein: R^{6'} to R^{9'} are independently selected from H, C₁-C₆ alkyl, C₁-C₆ alkoxy, C₁-C₁₂ alkoxyalkyl, SR¹², COOR¹² and N(R¹²)₂; and,
each R¹² is independently selected from C₁-C₆ alkyl or C₆-C₁₈ aryl,
subject to the proviso that n of radicals R^{6'} to R^{9'} are -R^{b}OC(O)C(R¹³)=CH₂ wherein:
R¹³ is H or C₁ alkyl;
n is an integer of from 1 to 3, preferably 1 or 2; and,
for each of said n radicals, R^{b} is independently selected from a covalent bond, C₂-C₁₂ alkylene, C₃-C₁₈ cycloalkylene or C₆-C₁₈ arylene,
said method comprising the steps of:
iii) reacting a disulfide compound of Formula (I) with a hydroxyl functional compound of Formula (II) to yield a compound of Formula (III) 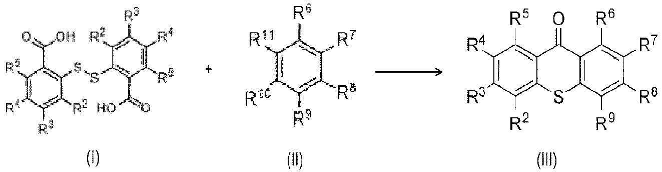 wherein: said reaction is performed in an acidic medium having a pH at or below 4.0;
R² to R⁵ are independently selected from H or C₁-C₆ alkyl;
R⁶ to R⁹ correspond to said substituents R^{6'} to R^{9'} and are independently selected from H, C₁-C₆ alkyl, C₁-C₆ alkoxy, C₁-C₁₂ alkoxyalkyl, SR¹², COOR¹² and N(R¹²)₂;
R¹⁰ and R¹¹ are H; and,
each R¹² is independently selected from C₁-C₆ alkyl or C₆-C₁₈ aryl,
subject to the proviso that n of radicals R⁶ to R⁹ are R^{b}(OH) wherein, for each of said n radicals, R^{b} is independently selected from a covalent bond, C₂-C₁₂ alkylene, C₃-C₁₈ cycloalkylene or C₆-C₁₈ arylene; and,
iv) reacting in an inert aprotic solvent said compound of Formula (III) with a compound of Formula (IV) to yield said compound of Formula (V) 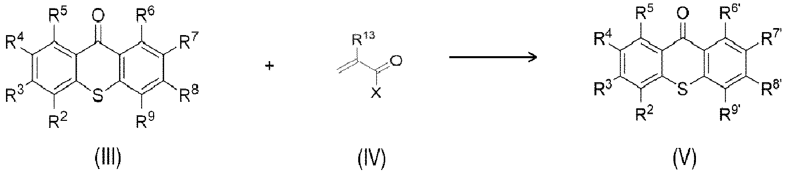 wherein: X is halide or -OC(O)C(R¹³)=CH₂; and,
the number of moles of (meth)acrylate groups provided by said compound of Formula (IV) is at least equimolar with the number of moles of hydroxyl groups provided by the compound of Formula (III).

2. The method according to claim 1, wherein R² to R⁵ are independently selected from H or C₁-C₄ alkyl.

3. The method according to claim 2, wherein R² to R⁵ are independently selected from H or C₁-C₂ alkyl.

4. The method according to claim 3, wherein R² to R⁵ are each H.

5. The method according to any one of claims 1 to 4, wherein in Formula (II):
R⁶ to R⁹ are independently selected from H, C₁-C₄ alkyl, C₁-C₄ alkoxy, C₁-C₈ alkoxyalkyl, COOR¹² and N(R¹²)₂;
R¹⁰ and R¹¹ are H; and,
each R¹² is independently selected from C₁-C₄ alkyl or C₆-C₁₈ aryl,
subject to the proviso that n of radicals R⁶ to R⁹ are -OH, wherein n is an integer of 1 or 2.

6. The method according to any one of claims 1 to 5, wherein said acidic medium of step i) has a pH at or below 3.5, preferably at or below 3.0.

7. The method according to any one claims 1 to 6, wherein step i) is performed at a temperature below the boiling point of the acid medium.

8. The method according to any one of claims 1 to 7, wherein step i) is performed at a temperature of from 20°C to 120°C, preferably from 40 to 120°C and more preferably from 60 to 100°C.

9. The method according to any one of clams 1 to 8, wherein in step ii) the molar ratio of (meth)acrylate groups provided by said compound of Formula (IV) to hydroxyl groups provided by said compound of Formula (III) is from 1:1 to 2:1, preferably from 1.1:1 to 1.5:1 and more preferably from 1.1:1 to 1.4: 1.

10. The method according to any one of claims 1 to 9, wherein X is halide and preferably wherein X is chloride.

11. The method according to claim 10, wherein the reaction of step ii) is performed in the presence of a base.

12. The method according to claim 11, wherein said base is selected from the group consisting of: tri(C₁-C₁₂)alkyl amines; di(C₁-C₁₂)alkyl(C₃-C₈)cycloalkyl amines; tri(C₁-C₁₀)alkenyl amines; and, mixtures thereof.

13. The method according to claim 11, wherein said base is selected from the group consisting of: trimethylamine; ethyldimethylamine; diethylmethylamine; triethylamine; triisopropylamine; tri-n-propylamine; tri-n-butylamine; tri-sec-butylamine; dibutylpentylamine; n-butyl-octyl-sec-butylamine; tripentylamine; trihexylamine; and, mixtures thereof.

14. The method according to any one of claims 1 to 13, wherein step ii) is performed at a temperature of from -40 to 20°C, preferably from -20 to 20°C.

15. The method according to claim 1 of preparing a compound of Formula (VA): 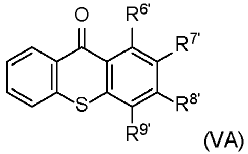 wherein: R^{6'} to R^{9'} are independently selected from H, C₁-C₄ alkyl, C₁-C₄ alkoxy, C₁-C₈ alkoxyalkyl, COOR¹² and N(R¹²)₂; and,
each R¹² is independently selected from C₁-C₄ alkyl or C₆-C₁₈ aryl,
subject to the proviso that n of radicals R^{6'} to R^{9'} are -OC(O)C(R¹³)=CH₂ wherein R¹³ is H or C₁ alkyl and further wherein n is an integer of 1 or 2,
said method comprising the steps of:
iii) reacting a disulfide compound of Formula (IA) with a hydroxyl functional compound of Formula (II) to yield a compound of Formula (IIIA) 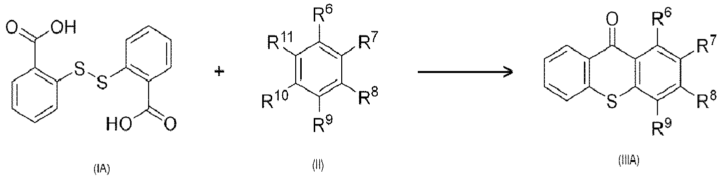 wherein:
said reaction is performed in an acidic medium having a pH at or below 3.0;
said reaction is performed at a temperature below the boiling point of the acidic medium and in the range from 40 to 120°C;
R⁶ to R⁹ correspond to said substituents R^{6'} to R^{9'} and are independently selected from the H, C₁-C₄ alkyl, C₁-C₄ alkoxy, C₁-C₈ alkoxyalkyl, COOR¹² and N(R¹²)₂;
R¹⁰ and R¹¹ are H; and,
each R¹² is independently selected from C₁-C₄ alkyl or C₆-C₁₈ aryl,
subject to the proviso that n of radicals R⁶ to R⁹ are -OH, wherein n is an integer of 1 or 2;
iv) reacting in an inert aprotic solvent said compound of Formula (IIIA) with a compound of Formula (IV) to yield said compound of Formula (VA) 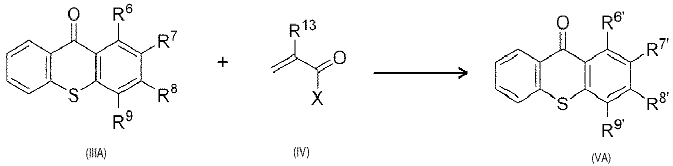
wherein: X is chloride;
the molar ratio of (meth)acrylate groups provided by said compound of Formula (IV) to hydroxyl groups provided by said compound of Formula (IIIA) is from 1.1:1 to 1.5:1; and,
the reaction of step ii) is performed in the presence of a base selected from the group consisting of: tri(C₁-C₁₂)alkyl amines; di(C₁-C₁₂)alkyl(C₃-C₈)cycloalkyl amines; tri(C₁-C₁₀)alkenyl amines; and, mixtures thereof.

16. Use of a compound of Formula (V) obtained in accordance with the method of any one of claims 1 to 15 as a monomer in a free-radical polymerization.

17. A copolymer obtained by free radical polymerization, said copolymer comprising, based on the total weight of monomers:
from 0.1 to 10 wt.% of a) at least one compound of Formula (V) obtained in accordance with the method of any one of claims 1 to 15; and,
from 90 to 99.9 wt.% of b) at least one ethylenically unsaturated non-ionic monomer which does not bear an epoxide group or a moiety decomposable under photo-irradiation to form a radical.
